# EUROPEAN PATENT APPLICATION

(11) **EP 3 603 533 A1**
(43) Date of publication of application: **05.02.2020**
(21) Application number: 18776959.1
(22) Date of filing: 30.03.2018
(51) Int. Cl.: A61B 17/00, A61N 7/00

(54) **ULTRASONIC IRRADIATION DEVICE**

(30) Priority: 30.03.2017 JP 2017069317
(71) Applicant: Kyocera Corporation, Kyoto-shi, Kyoto 612-8501 (JP)
(72) Inventor: TAKANO, Yasuju, Kyoto-shi Kyoto 612-8501 (JP)
(74) Representative: Viering, Jentschura & Partner mbB Patent- und Rechtsanwälte
(86) International application number: PCT/JP2018/013979
(87) International publication number: WO 2018/182018

(57) **Abstract**

An ultrasonic irradiation device according to the disclosure includes: an ultrasonic transducer for external application of focused ultrasound to a body surface; and a tenderness level meter comprising a pressing element that presses the body surface, a pressing unit that presses the pressing element against the body surface, a load measuring unit that measures a pressing force exerted on the body surface by the pressing element, and a pressure recorder for tenderness perception that records a pressing force at which a pressing action of the pressing element causes pain perception. This makes it possible to measure a tenderness level of an ultrasonic irradiation target area without moving an HIFU transducer (1) from above an affected area.

## Description

### Technical Field

The present disclosure relates to an ultrasonic irradiation device for external application of ultrasound waves to a painful body area for ablation.

### Background Art

As a way to alleviate ache and pain arising from metastatic bone cancer, joint pain, etc., there has been non-invasive bone-pain relief treatment to ablate a painful body area by external application of high-intensity focused ultrasound (hereafter referred to as "HIFU") to an affected area using an ultrasonic irradiation device (refer to Patent Literatures 1 and 2).

### Citation List

### Patent Literature

Patent Literature 1: Japanese Examined Patent Publication JP-B2 7-38858 (1995)
Patent Literature 2: Japanese Examined Patent Publication No. JP-B2 5429822

### Summary of Invention

An ultrasonic irradiation device according to the present disclosure includes: an ultrasonic transducer for external application of focused ultrasound to a body surface; and a tenderness level meter including a pressing element that presses the body surface, a pressing unit that presses the pressing element against the body surface, a load measuring unit that measures a pressing force exerted on the body surface by the pressing element, and a pressure recorder for tenderness perception that records a pressing force at which a pressing action of the pressing element causes pain perception.

### Brief Description of Drawings

Other and further objects, features, and advantages of the invention will be more explicit from the following detailed description taken with reference to the drawings wherein:
FIG. 1 is a schematic configuration diagram showing an ultrasonic irradiation section of an ultrasonic irradiation device according to an embodiment in the process of carrying out ultrasonic irradiation;
FIG. 2 is a schematic configuration diagram showing the operation of a pressure pain level meter in the ultrasonic irradiation device according to the embodiment; and
FIG. 3A is a front view of an ultrasonic irradiation portion of the ultrasonic irradiation device as seen from below, and FIG. 3B is a side view thereof.

### Description of Embodiments

Currently-used ultrasonic irradiation equipment designed for bone-pain relief treatment is classified into two types according to the manner of diagnosis and guide for an ultrasonic irradiation target area such as an area to be treated, namely (1) an apparatus with an MRI (Magnetic Resonance Imaging)-guided system called "MRgHIFU" and (2) an apparatus with an ultrasound-guided system called "USgHIFU". Both of them employ a therapeutic HIFU transducer for ablation of painful areas. There are HIFU transducers of various shapes, including one having a circular or rectangular contour, the surface of which is curved concavely in a direction opposite to the direction of ultrasonic irradiation, and one having a plate-like form with no curved surface.

In each of the MRgHIFU and USgHIFU, a tissue coagulation at a HIFU-treated area, viz., the ultrasonic irradiation target area within patient's body is monitored with the aid of images obtained by the MRI-guided system or the ultrasound-guided system, and, the monitoring result is used as the standard by which the outcome or effectiveness of ablation treatment is evaluated.

Unfortunately, currently-used typical diagnostic MRgHIFU and USgHIFU are each expensive, and its introduction is not so easy. Furthermore, for identification of an area of bone pain at moderate depths ranging from about a few to 30 millimeters below the body surface, heretofore it has been customary for a doctor to locate a point of tenderness on pressure by manual palpation. In this case, sensory evaluation is conducted by the patient on his or her own or by medical professionals according to a predetermined scale such as NRS (Numerical Rating Scale), in consequence whereof there may result no agreement between the outcome of ablation treatment assessed by image analysis and that assessed by the patient on his or her own.

Meanwhile, as a pain diagnosis procedure currently used by medical professionals, there has been a method using "tenderness level" expressed in the unit N (Newton) as a measure of the degree of pain. More specifically, during palpation by a doctor or other, a digital or analog tenderness meter or a tenderness meter which serves also as a muscle hardness meter is pressed against patient's body, and, a record is made as to a pressure load at which the first sign of tenderness or pain appears, expressed differently, a pressure high enough for the patient to feel pain, which is referred to simply as the tenderness level. In this connection, there is a conceivable method to evaluate ablation treatment outcomes using such a tenderness level as the standard for evaluation of treatment outcomes or effectiveness. More specifically, a transducer for ablation is moved away from above an ultrasonic irradiation target area subjected to treatment every time HIFU irradiation is carried out, so that the tenderness meter or the like can be pressed against the affected area which has already been irradiated with ultrasound to record the tenderness level.

However, to conduct such an evaluation after each and every ultrasonic irradiation, much time is required for repeated cycles of moving the transducer for ablation away from the affected area and returning the transducer to the same position after tenderness level measurement as described above. Compounding the problem, patients face heavy psychological burdens such as an increase in feelings of anxiety about diagnostic measurement.

In any case, monitoring of pain-relief ablation treatment outcomes during HIFU treatment costs time and money, and places burdens on patients under the current circumstances.

The present disclosure has been made in view of the circumstances as discussed supra, and accordingly its object is to provide an ultrasonic irradiation device that achieves effective ultrasonic irradiation by efficient acquisition of a tenderness level on pressure of an affected area.

The following describes an embodiment of the present disclosure.

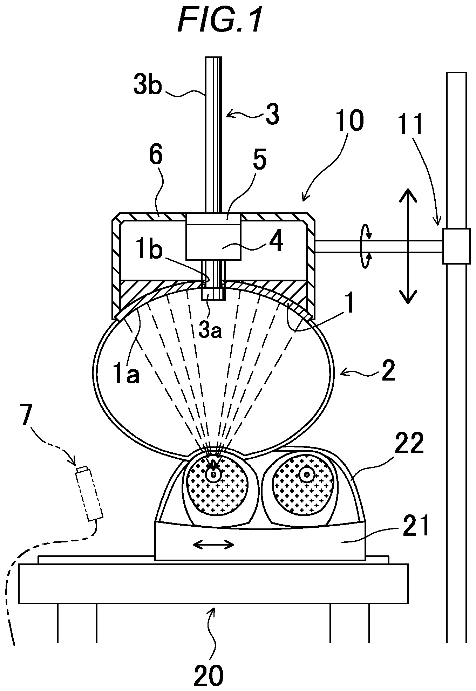

An ultrasonic irradiation device according to this embodiment is an ultrasonic irradiation device designed for bone-pain relief treatment, which is used to alleviate bone pain from metastatic bone cancer, joint pain, etc., in other words, ache and pain occurring at moderate depths ranging from about a few millimeters to 30 mm below the body surface. The ultrasonic irradiation device includes: an ultrasonic transducer for external application of focused ultrasound to a body surface; and a tenderness level meter including a pressing element that presses the body surface, a pressing unit that presses the pressing element against the body surface, a load measuring unit that measures a pressing force exerted on the body surface by the pressing element, and a pressure recorder for tenderness perception that records a pressing force at which the pressing action of the pressing element causes pain perception. As shown in FIG. 1, the ultrasonic irradiation device includes a HIFU irradiation section 10 including a single ultrasound emitter or a plurality of ultrasound emitters and a treatment support table 20, and further includes a power supply section, a coolant producing section, and a control section such as a personal computer, which are not shown.

A movable bed 21 on the treatment support table 20 and a support 11 for supporting the HIFU irradiation section 10 are movable in the directions of two plane coordinate axes, namely X-axis direction and Y-axis direction, and in the direction of a height coordinate axis, namely Z-axis direction, respectively, so that the position of patient's body lying in the bed under ultrasonic irradiation treatment can be freely adjusted. This makes it possible to hold an ultrasonic irradiation target area, such as the thighs of both legs of the patient in this case, in a proper position immediately below the HIFU irradiation section 10 in a vertical direction.

The HIFU irradiation section 10 may be made rotatable about the axis of the support 11 so as to apply ultrasound waves obliquely relative to the vertical direction. Moreover, the movable bed 21 may be equipped with a holding strap 22, etc. for retaining the posture and position of the patient. Mechanisms for moving and holding part of patient's body subjected to ultrasonic irradiation are not limited to those as described above, and, it is possible to use any heretofore known patient-supporting and moving mechanism that is adopted for use in other medical equipment on account of its capability of holding an ultrasonic irradiation target area in a proper position.

In the ultrasonic irradiation device according to this embodiment, the pressing element is shaped in a rod, and, the ultrasonic transducer includes an ultrasonic irradiation portion including a surface opposed to an irradiation target. The ultrasonic irradiation portion is provided with a through hole opening in the surface opposed to the irradiation target, and the pressing element is inserted into the through hole. The pressing element is movable through the through hole. Moreover, in the ultrasonic irradiation device according to this embodiment, the ultrasonic transducer includes a single ultrasonic emitter or a plurality of ultrasound emitters located on the surface thereof opposed to the irradiation target.

More specifically, the HIFU irradiation section 10 as shown in FIG. 1 mainly includes: an ultrasonic transducer 1, which serves as an ultrasonic irradiation portion, shaped in a circular plate so as to spread out above the affected area; a resin film-made balloon 2 that receives therein cooling water for cooling part of patient's body subjected to ultrasonic irradiation which is the ultrasonic irradiation target area; and a housing 6 built as an irradiation portion case that enables the ultrasonic transducer 1 and the balloon 2 to be suspended above patient's body, and is movably supported by the support 11.

In the housing 6, as constituent components of a tenderness level meter including a pressure recorder for pressure pain perception, there are disposed a rod-like pressing element 3, a pressing element-driving portion 4, which serves as a pressing unit, that presses the pressing element 3 against the body surface, and a load meter 5, which serves as a load measuring unit, that measures a pressing force exerted on the body surface by the pressing element 3. Each of the components and device constituting the tenderness level meter does not necessarily have to be disposed in a unitary housing. Moreover, mutual functional complement or functional integration allows omission of some of the components and device from the construction. For example, a recorder used as the pressure recorder for tenderness perception that records a pressing force exerted by the pressing element 3, expressed differently, a peak load value, may be incorporated in the load meter 5, or, the earlier described control section may be designed to serve also as the pressure recorder for tenderness perception. Note that means for providing connection between the constituent members, such as piping and wiring, is not shown in each drawing.

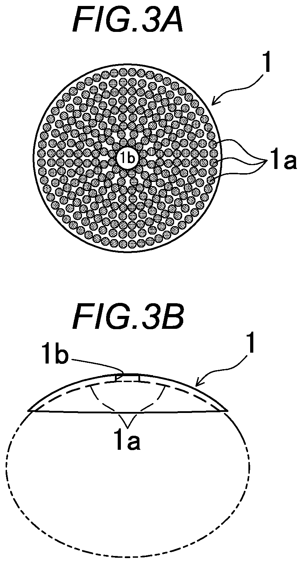

The constituent members of the HIFU irradiation section 10 will be described in more detail. As shown in FIG. 3B, the ultrasonic transducer 1 is in the general form of a circular plate or a disk shaped so as to spread out above the affected area. The lower surface of the ultrasonic transducer 1 facing toward the affected area is curved concavely in a vertical downwards direction to define a parabolic surface. As shown in FIG. 3A, on the inner side of the concave surface, many ultrasound emitters 1a are disposed. Moreover, the center of the discotic and parabolic form is provided with a through hole 1b as an opening capable of inserting the pressing element 3 of the tenderness level meter as described later.

The inner surface of the ultrasonic transducer 1 is curved with a radius of curvature such that ultrasound waves can converge to a point. Thus, when the individual ultrasound emitters 1a oscillate simultaneously, as shown in FIG. 1, resultant ultrasound emission is focused to one point, with consequent production of high-intensity HIFU.

The ultrasonic transducer 1 is, as exemplified, shaped in a circular plate or the like so as to spread out above the affected area. This is intended to arrange as many ultrasound emitters as possible on a wide surface opposed to the affected area to ensure high-intensity ultrasound emission. Thus, the general form of the ultrasonic irradiation portion is not limited to the circular plate or disk, and, it is possible to use any other shape having a surface wide enough to face toward the affected area without special limitation. For example, the ultrasonic irradiation portion may be given a circular or rectangular contour, the surface of which is curved concavely in a direction opposite to the direction of ultrasonic irradiation, or may be shaped in a plate having no curve.

Moreover, as the ultrasound emitters, phased-array HIFU emitters capable of adjusting an ultrasound focusing point and a depth of field may be used. This makes it possible to change the radius of curvature of the parabolic form of the ultrasonic transducer 1, as well as to adopt a flat-shaped irradiation portion having a flat lower surface.

The balloon 2 is constructed of a resin-made film. Degassed cold water produced by the coolant producing section, which is not shown, such as cold water of a temperature of about 18°C in this case, is passed in circulation into the balloon 2 from the coolant producing section. This makes it possible to keep cooling water temperature in the balloon 2 constant, and thereby cool the body surface contacted by the bottom of the lower part of the balloon 2. The reason for cooling the body surface is to reduce heat-caused-injury or burn in the body surface and body's superficial part under ultrasonic irradiation. Moreover, the cooling water is circulated in a degassed state to reduce generation of air bubbles due to ultrasonic irradiation, for example.

The pressing element 3 of the tenderness level meter including the pressure recorder for tenderness perception is in the general form of a circular cylinder. A discotic pressing plate 3a which abuts on the body surface is attached to the front end, viz., the lower end as seen in the drawing, of the pressing element 3. The pressing plate 3a is inserted into the ultrasonic transducer 1 through the through hole 1b of the ultrasonic irradiation portion. Moreover, a cylindrical portion 3b is mounted for vertical reciprocating up-and-down motion via the pressing element-driving portion 4 and the load meter 5 as described later, and, the pressing plate 3a-side lower end of the cylindrical portion 3b is inserted into the ultrasonic transducer 1.

A sealing member such as an O ring is disposed for making a gap between the cylindrical portion 3b and the surrounding through hole 1b watertight to prevent leakage of the degassed cooling water. The water leakage through the through hole 1b can be prevented by other means. Moreover, the form of the pressing element 3 is given by way of example, and, the pressing element 3 may be given any other form capable of pressing the body surface through the bottom of the interior of the balloon 2.

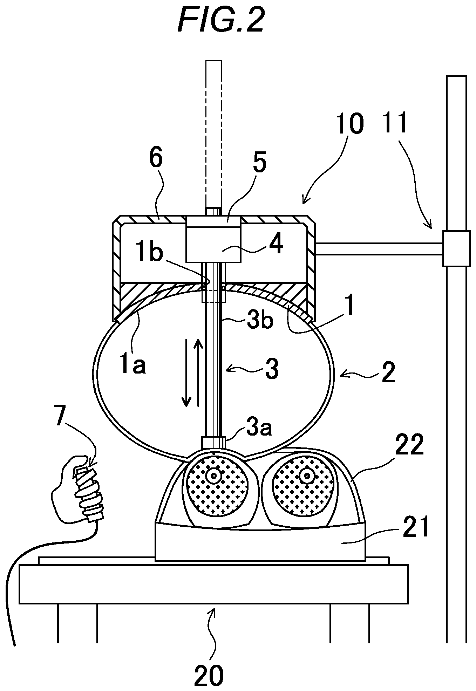

The pressing element-driving portion 4 serving as the pressing unit of the tenderness level meter is configured to impart vertical motion to the pressing element 3 via a driving source such as a motor or compressed air. As shown in FIG. 2, in the pressing element-driving portion 4, during intervals when ultrasound emission is not carried out by the ultrasonic transducer 1, the pressing plate 3a of the pressing element 3 is pressed against the body surface corresponding to the ultrasonic irradiation target area to cause tenderness on pressure at the painful area. Moreover, the load meter 5 or a force gauge is attached to the pressing element-driving portion 4 so that a pressing force exerted on the body surface by the pressing element 3 can be measured and gauged.

The pressing element-driving portion 4 exemplifies a moving portion in the present disclosure. That is, as will be described hereafter, for example, the pressing element-driving portion 4 functions as a moving portion that moves the pressing element 3 when it is desired to raise the pressing element 3 to a retracted position near the through hole 1b of the ultrasonic transducer 1 away from the body surface at the time of ultrasonic irradiation or following the completion of tenderness level measurement. The moving portion and the pressing unit of the tenderness level meter may either be, as exemplified, implemented by the sharing of a single device or system for pressing action and moving action or be implemented by a combination of a device or system constituting the pressing unit and another device or system constituting the moving portion.

As described above, the load meter 5 serving as the load measuring unit may include a recorder that records a peak load value as a pressing force exerted by the pressing element 3. Moreover, the driving source or driving mechanism for the pressing element-driving portion 4 is not limited to specific means.

Moreover, as shown in FIG. 2, the loadmeter 5 may include a switch 7 which serves as a pain notification unit that notifies others of pain arising from the pressing force exerted by the pressing element 3 of the tenderness level meter.

That is, it is preferable that the ultrasonic irradiation device according to this embodiment employs a design wherein the tenderness level meter includes a pain notification unit that notifies others of the pain arising from the pressing force exerted by the pressing element, as well as a design wherein the tenderness level meter includes a pressing stop portion that causes the pressing element to stop pushing against the body surface.

In operating the ultrasonic irradiation device thereby constructed, as the first step, a sign or mark is put on part of the body surface which has been assumed to be the best irradiation position on the basis of data obtained by, for example, prior doctor's palpation or examination using an MRI-guided system or an ultrasound-guided system with a marking pen or the like.

Next, with reference to the sign or mark on the body surface, a medical professional worker such as a doctor moves patient's body lying supine on the movable bed 21 of the treatment support table 20 to a proper position for irradiation by adjusting the positions of the movable bed 21 and the HIFU irradiation section 10 moving in the three-axis directions, and then lowers the HIFU irradiation section 10 toward the patient, with the top of the main irradiation portion of the HIFU irradiation section 10 focused on the sign or mark.

After making a decision about the HIFU irradiation position, in accordance with a treatment plan developed in advance, the affected area is irradiated with HIFU for a predetermined period of time, for example, for 0.1 to a few seconds under normal conditions, for ablation to induce inactivation effect. At this time, the rod-like pressing element 3 of the tenderness level meter is raised to the retracted position near the through hole 1b of the ultrasonic transducer 1 so as not to obstruct HIFU irradiation.

Then, while the HIFU irradiation section 10 is lowered in the position above the patient, based on a command signal from the control section, the pressing element 3 of the tenderness level meter is lowered to press the pressing plate 3a of the pressing element 3 against the body surface, with the bottom of the balloon 2 lying in between. At this time, as shown in FIG. 2, the patient under tenderness evaluation takes hold of the earlier described switch 7 to complain about pain or tenderness arising from the pressing force exerted by the pressing element 3 whenever the pain or tenderness reaches an intolerable level. Alternatively, it is possible to use a system in which the patient is able to complain about pain by releasing his or her hand from a button or the like.

Next, with the pressing element 3 kept in contact with the body surface and the bottom of the balloon 2, the pressing element 3 is pressed against the body surface via the pressing element-driving portion 4, during which period a pressure load or pressing force is measured by the load meter 5. When a to-be-treated person finds the pressure-caused-pain intolerable, the patient presses a button or the like of the switch 7 in the pain notification unit, whereupon the value of a pressure peak at that point of time is recorded, and, further pressing action of the pressing element 3 is stopped.

Then, the pressing element 3 is driven to move upward by the pressing element-driving portion 4, and, when the pressing element 3 reaches the retracted position near the through hole 1b of the ultrasonic transducer 1, one tenderness level measurement comes to an end. It is desirable to take about three consecutive measurements of tenderness level in the time interval between ultrasonic irradiation process steps, and average the measured values.

Thus, the ultrasonic irradiation device according to this embodiment can quickly measure a tenderness level for an ultrasonic irradiation target area which is hidden below the ultrasonic transducer 1 and the balloon 2 without the necessity of moving the therapeutic ultrasonic transducer 1 away from above the affected area or adjusting the position of the ultrasonic transducer 1. This makes it possible to reduce ultrasonic irradiation time for ultrasonic ablation treatment, and thereby reduce burdens on patients.

Besides, the outcome of pain-relief treatment by ultrasonic ablation can be obtained right away, and information on the outcome can be fed back to the very next ultrasonic irradiation. This makes it possible to protect patients from excessive ultrasonic irradiation and unduly-painful extra tenderness level measurement, and thereby eliminate patient anxiety about pain.

That is, in the ultrasonic irradiation device according to this embodiment, a tenderness level on pressure of an affected area is efficiently acquired with high reproducibility, and, easily obtainable outcomes of pain-relief ultrasonic ablation treatment can be exploited in or fed back to the next ultrasonic irradiation. This makes it possible to complete ablation treatment with ultrasonic irradiation effectively in a short period of time, and thereby reduce burdens on patients under ultrasonic irradiation treatment.

Moreover, by designing the switch 7 to serve also as a pressing stop portion that causes the rod-like pressing element 3 to stop pushing against the body surface immediately as required, or serve also as an emergency stop portion that stops ablation treatment by ultrasound emission from the ultrasonic transducer 1 immediately as required, it is possible to minimize patient anxiety about a fear of pain entailed by ablation or tenderness level measurement.

Tenderness measurement using the tenderness level meter does not necessarily have to be carried out every time one ultrasonic irradiation is completed. Moreover, although the through hole 1b for inserting the pressing element 3 of the tenderness level meter is formed at the center of the discotic ultrasonic transducer 1 in the above-described embodiment, the through hole 1b may be formed in any other location of the discotic ultrasonic transducer as long as the front end, viz., the lower end of the pressing element 3 can reach the ultrasonic irradiation target area.

Moreover, for example, when the affected area can be observed or exposed to view without the necessity of moving the therapeutic ultrasonic transducer 1 away from above the ultrasonic irradiation target area during ultrasound application from the lateral side of patient's body, there is no need for the pressing element 3 to pass interiorly through the balloon 2. In such a case, the tenderness level meter may be disposed on the outer side of the discotic ultrasonic transducer 1.

The form of the switch 7 serving as a pain notification unit is not limited to the exemplification, and, it is possible to use any other patient-friendly form, such as a button or a touch panel, without special limitation. Moreover, a plurality of switches 7 may be disposed within the reach of patients.

That is, one of examples of the ultrasonic irradiation device according to the embodiment which includes the pain notification unit can readily notify medical professionals that pain or tenderness arising from the pushing action of the pressing element has reached an intolerable level before the patient complains the pain or tenderness in words.

Moreover, one of examples of the ultrasonic irradiation device which includes the pressing stop portion can bring the tenderness level meter to an emergency stop whenever the patient has feelings of anxiety about the operation of the tenderness level meter. This makes it possible to further reduce psychological burdens on patients, such as patient anxiety about a fear of pain entailed by tenderness level measurement, and therefore it is preferable that the ultrasonic irradiation device includes the pressing stop portion.

No special structural limitation is imposed on the constituent components of the ultrasonic irradiation device, except for the HIFU irradiation section 10, and, the disclosure is broadly applicable to HIFU irradiation system-equipped ultrasonic irradiation devices.

Moreover, the disclosure may be embodied in other specific forms without departing from the spirit or essential characteristics thereof. The present embodiments are therefore to be considered in all respects as illustrative and not restrictive, the scope of the disclosure being indicated by the appended claims rather than by the foregoing description and all changes which come within the meaning and the range of equivalency of the claims are therefore intended to be embraced therein.

### Reference Signs List

- 1:: Ultrasonic transducer
1a: Ultrasound emitter
1b: Through hole
- 2:: Balloon
- 3:: Pressing element
3a: Pressing plate
3b: Cylindrical portion
- 4:: Pressing element-driving portion
- 5:: Load meter
- 6:: Housing
- 7:: Pain notification unit (switch)
- 10:: HIFU irradiation section
- 11:: Support
- 20:: Treatment support table
- 21:: Movable bed
- 22:: Holding strap

## Claims

1. An ultrasonic irradiation device, comprising:
an ultrasonic transducer for external application of focused ultrasound to a body surface; and
a tenderness level meter comprising a pressing element that presses the body surface, a pressing unit that presses the pressing element against the body surface, a load measuring unit that measures a pressing force exerted on the body surface by the pressing element, and a pressure recorder for tenderness perception that records a pressing force at which a pressing action of the pressing element causes pain perception.

2. The ultrasonic irradiation device according to claim 1, wherein the tenderness level meter comprises a moving portion that moves the pressing element to a position for contact with the body surface and to a position away from the body surface.

3. The ultrasonic irradiation device according to claim 1 or 2, wherein the pressing element is shaped in a rod,
the ultrasonic transducer comprises an ultrasonic irradiation portion comprising a surface opposed to an irradiation target,
the ultrasonic irradiation portion is provided with a through hole opening in the surface opposed to the irradiation target, and the pressing element is inserted into the through hole, and
the pressing element is movable through the through hole.

4. The ultrasonic irradiation device according to claim 3, wherein the ultrasonic transducer comprises a single ultrasound emitter or a plurality of ultrasound emitters located on the surface opposed to the irradiation target.

5. The ultrasonic irradiation device according to any one of claims 1 to 4, wherein the tenderness level meter comprises a pain notification unit that notifies others of pain generation from a pressing force exerted by the pressing element.

6. The ultrasonic irradiation device according to any one of claims 1 to 5, wherein the tenderness level meter comprises a pressing stop portion that causes the pressing element to stop pushing against the body surface.
